(19) **Europäisches Patentamt**

**European Patent Office**

**Office européen des brevets**

(11) **EP 1 064 409 B1**

(12) **EUROPÄISCHE PATENTSCHRIFT**

(45) Veröffentlichungstag und Bekanntmachung des Hinweises auf die Patenterteilung:
**20.11.2002 Patentblatt 2002/47**

(21) Anmeldenummer: **99920549.5**

(22) Anmeldetag: **16.03.1999**

(51) Int Cl.[7]: **C12Q 1/68**, G01N 21/64

(86) Internationale Anmeldenummer:
**PCT/DE99/00729**

(87) Internationale Veröffentlichungsnummer:
**WO 99/047702 (23.09.1999 Gazette 1999/38)**

(54) **VERFAHREN UND VORRICHTUNG ZUM IDENTIFIZIEREN EINER MARKIERUNG**

METHOD AND DEVICE FOR IDENTIFYING A TAG

PROCEDE ET DISPOSITIF D'IDENTIFICATION D'UN MARQUAGE

(84) Benannte Vertragsstaaten:
**AT BE CH DE DK ES FR GB IT LI NL**

(30) Priorität: **18.03.1998 DE 19811730**

(43) Veröffentlichungstag der Anmeldung:
**03.01.2001 Patentblatt 2001/01**

(73) Patentinhaber: **november Aktiengesellschaft Gesellschaft für Molekulare Medizin**
**91056 Erlangen (DE)**

(72) Erfinder: **BERTLING, Wolf**
**D-91056 Erlangen (DE)**

(74) Vertreter: **Gassner, Wolfgang, Dr.**
**Patentanwalt**
**Nägelsbachstrasse 49a**
**91052 Erlangen (DE)**

(56) Entgegenhaltungen:
EP-A- 0 668 498    EP-A- 0 745 690
EP-A- 0 861 906    EP-A- 0 909 823
WO-A-97/46707    WO-A-97/46708
US-A- 4 996 143    US-A- 5 607 834
US-A- 5 723 591

• WOUDENBERG T M ET AL: "QUANTITATIVE PCR BY REAL TIME DETECTION" PROCEEDINGS OF THE SPIE, Bd. 2680, 1. Januar 1996 (1996-01-01), Seiten 306-315, XP000197422
• MORRISON L E ET AL.: "Solution-phase detection of polynucleotides using interacting fluorescent labels and competitive hybridization" ANALYTICAL BIOCHEMISTRY, Bd. 183, 1989, Seiten 231-244, XP002113097
• CARDULLO R A ET AL: "DETECTION OF NUCLEIC ACID HYBRIDIZATION BY NONRADIATIVE FLUORESCENCE RESONANCE ENERGY TRANSFER" PROCEEDINGS OF THE NATIONAL ACADEMY OF SCIENCES OF USA, Bd. 85, Nr. 23, 1. Dezember 1988 (1988-12-01), Seiten 8790-8794, XP000453537 ISSN: 0027-8424 in der Anmeldung erwähnt

SEQUENZPROTOKOLLE

<110> november AG Novus Medicatus Bertling Gsellschaft für Molekulare Medizin

<120> Verfahren und Vorrichtung zum Identifizieren einer Markierung

<130> 380293ga5

<140>
<141>

<160> 2

<170> PatentIn Ver. 2.1

<210> 1
<211> 32
<212> DNA
<213> human

<400> 1

```
ccaagcctgg agggatgata ctttgcgctt gg                              32
```

<210> 2
<211> 32
<212> DNA
<213> human

<400> 2

```
ccaagcgcaa agtatcatcc ctccaggctt gg                              32
```

## Beschreibung

Die Erfindung betrifft ein Verfahren und eine Vorrichtung zum Identifizieren einer an einem Festkörper vorgesehenen eine erste Nukleotidsequenz aufweisenden Markierung.

Aus der US 4,996,143 ist ein Verfahren bekannt, bei dem ein erster und ein zweiter Primer in einem Abstand von 2 bis 7 Nukleotiden an die nachzuweisende Nukleotidsequenz gebunden werden. Der erste und der zweite Primer sind jeweils mit einem fluorophoren Molekül versehen. Im Bindungszustand kommt es infolge des Förster-Effekts zu einem strahlungslosen Energieübergang vom einem fluorophoren Molekül auf das andere. Das bewirkt eine spezifische Fluoreszenz.

Aus der US 5,607,834 ist es bekannt, zum Nachweis einer Nukleotidsequenz einen Primer mit einer Haarnadelschleife zu verwenden. Dabei sind an den Schleifenabschnitten der Haarnadelschleife gegenüberliegend ein fluorophores Molekül und ein Quencher vorgesehen. Der Abstand zwischen dem fluorophoren Molekül und dem Quencher ermöglichen einen strahlungslosen die Fluoreszenz löschenden Energieübergang. Wenn der Primer allerdings mit einem komplementären Gegenstrang hybridisiert, wird die Haarnadelschleife geöffnet. Die eine Fluoreszenz löschende räumliche Beziehung zwischen dem fluorophoren Molekül und dem Quencher wird geändert. Damit ist eine Fluoreszenz beobachtbar.

Die DE 195 81 489 T1 beschreibt ein Verfahren zur Identifizierung und zum Nachweis von Komponenten in einer Vielkomponenten-Mischung. Dabei wird ein Marker benutzt, der mindestens zwei an einem Grundgerüst gebundene fluorophore Gruppen in Energieübertragungsbeziehung aufweist. - Das bekannte Verfahren eignet sich für Trennungsverfahren, wie die Elektrophorese, die Chromatographie oder dgl.. Es eignet sich nicht für die Identifizierung einer an einer festen Phase vorgesehenen Markierung.

Aus Cardullo, R. A. u.a.; Detection of nucleic acid hybrdisation by nonradiative fluorescence resonance energy transfer; Proc. Natl. Acad. Sci. USA 85 (1988) 8790-8794 ist der Fluoreszenz-Energie-Transfer in Hybriden zwischen fluorophormarkierten Oligonukleotiden beschrieben. Die Nutzung des Fluoreszenz-Energie-Transfes zur Identifikation von Markierungen ist hier nicht erwähnt.

US-A-5,723,591 und L. E. Morrison et al. (1989) Analytical Biochemistry 183, 231-244 offenbaren die Anwendung von Fluorophor- und Quencher-markierten Oligonukleotidproben in einem Hybridisierungsassay zum Nachweis von spezifischen, nicht markierten Polynukleotiden. EP-A-0 909 823 (Stand der Technik gemäß Art. 54 (3) EPÜ und nur in Bezug auf Neuheit zu berücksichtigen), EP-A-0 668 498, T. M. Woudenberg & J. Stevens (1996) Proc.SPIE, vol. 2680, 306-315, WO-A-97/46708 und WO-A-97/46707 offenbaren Verfahren und Vorrichtungen zur Identifizierung eines Nukleotidsequenz auf der Basis einen durch Hybridisierungs modulierten Übertragung von Fluoreszenzenergie zwischen einem Donorfluorophor und einem Akzeptorfluorophor, die am Detektionsnukleotidsequenz gebunden sind. Keine dieser Dokumente offenbart ein Verfahren und/oder eine Vorrichtung, bei der beide Nukleotidsequenzen an einer festen Phase gebunden sind.

Die bekannten Verfahren sind nicht besonders sensitiv. Sie können nur in Lösung angewendet werden. Zur einfachen und schnellen Identifizierung von an einen Festkörper gebunden Nukleotidsequenzen sind sie nicht geeignet.

Aufgabe der vorliegenden Erfindung ist es, die Nachteile nach dem Stand der Technik zu beseitigen. Insbesondere sollen ein Verfahren und eine Vorrichtung angegeben werden, mit denen eine einfache und schnelle Identifizierung von einer an einem Festkörper vorgesehenen eine erste Nukleotidsequenz aufweisenden Markierung möglich ist. Die Identifizierung soll insbesondere trocken, d.h. ohne das Herstellen einer Lösung, möglich sein.

Diese Aufgabe wird durch die Merkmale der Ansprüche 1 und 8 gelöst. Zweckmäßige Ausgestaltungen ergeben sich aus den Merkmalen der Ansprüche 2 bis 7 und 9 bis 20.

Erfindungsgemäß ist ein Verfahren zum Identifizieren einer an einem Festkörper vorgesehenen Markierung, die eine erste mit ihrem einen Ende am Festkörper gebundene Nukleotidsequenz aufweist, wobei die erste Nukleotidsequenz mit einem Detektionsmittel in Kontakt gebracht wird, wobei das Detektionsmittel eine feste Phase aufweist, an die eine zur ersten Nukleotidsequenz korrespondierende zweite Nukleotidsequenz mit ihrem ersten Ende gebunden ist und wobei die zweite Nukleotidsequenz am zweiten Ende eine erste fluorophore Gruppe aufweist, wobei beim Inkontaktbringen die erste und die zweite Nukleotidsequenz hybridisieren, und wobei bei der Hybridisierung der ersten und zweiten Nukleotidsequenz eine Fluoreszenzreaktion ausgelöst wird. - Das ermöglicht eine einfache und schnelle Identifizierung einer an einem Festkörper vorgesehenen Nukleotidsequenz. Die Herstellung einer Lösung ist dazu nicht erforderlich.

Es kann vorteilhafterweise vor oder beim Inkontaktbringen eine Hybridisierung der ersten Nukleotidsequenz mit einer dritten Nukleotidsequenz gelöst werden.

Nach einem weiteren Ausgestaltungsmerkmal kann die erste Nukleotidsequenz beim Inkontaktbringen durch Magnetwirkung in Richtung des ersten Detektionsmittels bewegt werden. Das Inkontaktbringen kann auch durch Auftragen eines Gels oder einer, vorzugsweise viskosen, Flüssigkeit auf das Detektionsmittel und/oder die Markierung gefördert werden.

Zweckmäßigerweise wird vor dem Inkontaktbringen eine die erste Nukleotidsequenz überdeckende Schutzschicht entfernt, wobei die Markierung vorteilhafterweise auf eine Temperatur von mehr als 50°C erwärmt wird. Nach dem Inkontaktbringen wird die Markierung zweckmäßigerweise auf eine Temperatur von weniger als 50°C abgekühlt.

Gemäß der Erfindung ist desweiteren vorgesehen eine Vorrichtung zum Identifizieren einer an einem Festkörper vorgesehenen Markierung, die eine erste mit ihrem einen Ende am Festkörper gebundene Nukleotidsequenz aufweist, mit einem Detektionsmittel, das eine feste Phase aufweist, an die eine zur ersten Nukleotidsequenz korrespondierende zweite Nukleotidsequenz mit ihrem ersten Ende gebunden ist und am zweiten Ende eine erste fluorophore Gruppe aufweist, so dass beim Inkontaktbringen des Detektionsmittels mit der Markierung die erste und die zweite Nukleotidsequenz hybridisieren und bei der Hybridisierung der ersten und zweiten Nukleotidsequenz eine Fluoreszenzreaktion auslösbar ist. Eine solche Vorrichtung ermöglicht eine einfache und schnelle Identifizierung einer an einem Festkörper vorgesehenen Nukloetidsequenz. Die Herstellung einer Lösung ist dazu nicht erforderlich.

Vorteilhafterweise ist ein erstes Ende der ersten Nukleotidsequenz über Spacermoleküle kovalent an den Festkörper gebunden. Eine solche Markierung ist besonders dauerhaft.

An ein zweites Ende der ersten Nukleotidsequenz ist zweckmäßigerweise ein magnetisches Mittel gebunden. Dabei kann es sich um einen magnetischen Kunststoff, $Fe_2O_3$-Partikel oder dgl. handeln. Die Größe der magnetischen Mittel beträgt vorzugsweise 20nm bis 200nm Es können auch Kunststoffe Verwendung finden, die bereits ab einer Kettenlänge von 4 Monomeren superparamagnetisch sind. Aus solchen Kunststoffen hergestellte magnetische Mittel könne eine Größe von wenigen Angstöm aufweisen.'

Nach einem weiteren Ausgestaltungsmerkmal ist das erste Ende der zweiten Nukleotidsequenz über Spacermoleküle an die feste Phase gebunden. Das Detektionsmittel kann eine dritte Nukleotidsequenz aufweisen, die zumindest abschnittsweise zur zweiten Nukleotidsequenz korrespondierend ausgebildet ist. Ein erstes Ende der dritten Nukleotidsequenz kann ebenfalls über Spacermoleküle an die feste Phase gebunden sein.

Die Spacermoleküle weisen vorzugsweise eine Länge von 0,2 bis 1μ auf. Die feste Phase des Detektionsmittels kann aus einem lichtdurchlässigen Material, z.B. Kunststoff, Glas oder Quarz, hergestellt sein. Auf der Oberfläche der festen Phase kann ferner eine aus wasserbindenden Substanzen, z.B. Polyethylenglycol, Glyzerin oder dgl., hergestellte Schicht vorgesehen sein.

An einem zweiten Ende der dritten Nukleinsäure ist zweckmäßigerweise eine zweite fluorophore Gruppe oder ein Quencher vorgesehen. Die zweiten und die dritten Nukleinsäuresequenzen liegen bei der Identifizierung zweckmäßigerweise im hybridisierten Zustand vor, so dass eine Wechselwirkung zwischen dem ersten fluorophoren Molekül bzw. dem Quencher sich ausbildet. Die sich ausbildende Wechselwirkung bewirkt entweder eine Löschung einer Fluoreszenz des ersten fluorophoren Moleküls oder auch eine besonders starke Fluoreszenzreaktion infolge eines direkten oder strahlungslosen Energieübergangs vom ersten auf das zweite fluorophore Molekül oder umgekehrt. Sobald die räumliche Beziehung zwischen dem ersten fluorophoren Molekül und dem zweiten fluorophoren Molekül bzw. dem Quencher geändert wird, ist eine Änderung der Fluoreszenz beobachtbar. Die Änderung der Fluoreszenz dient zur Identifizierung der ersten Nukleotidsequenz.

Als besonders vorteilhaft hat es sich erwiesen, dass die erste, zweite und die dritte Nukleinsäuresequenz aus einer Desoxyribonukleinsäure (=DNA) oder peptischen Nukleinsäure (=PNA) gebildet sind. Andere, insbesondere Nukleaseresistente Nukleinsäurederivate, sind ebenfalls vorstellbar.

Das Detektormittel kann ferner eine Lichtquelle und/oder einen Fluoreszenzdetektor aufweisen. Es kann ferner mit einer Einrichtung zur Erzeugung eines Wärme-Pulses, vorzugsweise eines IR-Laser-, NMR-, Mikrowellen- oder Ultraschall-Pulses versehen sein. Dadurch kann die Markierung kurzzeitig aufgeheizt werden.

Ausführungsbeispiele der Erfindung werden anhand der Zeichnung näher erläutert. Hierin zeigen:

Fig. 1 eine schematische Darstellung einer Markierung,

Fig. 2 eine schematische Teilansicht eines Detektors,

Fig. 3 a-c die Wirkung eines Magnetfelds auf eine Markierung gemäß Fig. 1,

Fig. 4 eine schematische Teilansicht der Oberfläche des Detektors,

Fig. 5 die Markierung gemäß Fig. 1 im Kontakt mit dem Detektor gemäß Fig. 3,

Fig. 6 a-c den Detektionsvorgang,

Fig. 7 a, b eine zweite Markierung im nichthybridisierten und im hybridisierten Zustand,

Fig. 8 a, b eine dritte Markierung im nichthybridisierten und im hybridisierten Zustand,

Fig. 9 a, b      eine vierte Markierung im nichthybridisierten und im hybridisierten Zustand,

Fig. 10 a, b      eine fünfte Markierung im nichthybridisierten und im hybridisierten Zustand,

Fig. 11      die Fluoreszenz eines Detektornukleotids (1) ohne Markierungsnukleotid und (2) mit Markierungsnukleotid,

Fig. 12 a      einen mit Markierungsnukleotid beschichteten Objektträger und

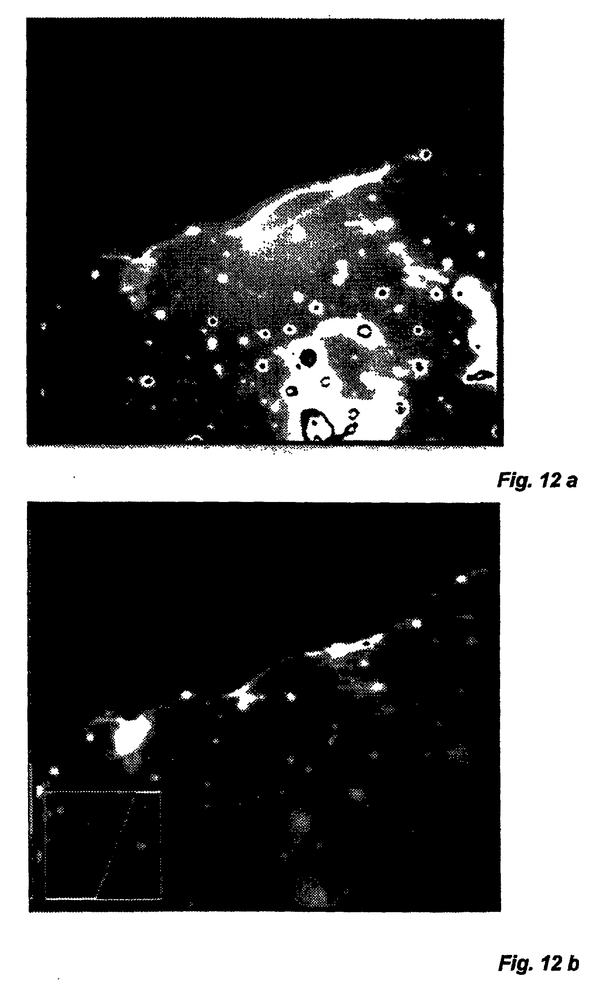

Fig. 12 b      das Markierungsnukleotid gemäß Fig. 12 a im Hybridisierungszustand mit einem Detektornukleotid.

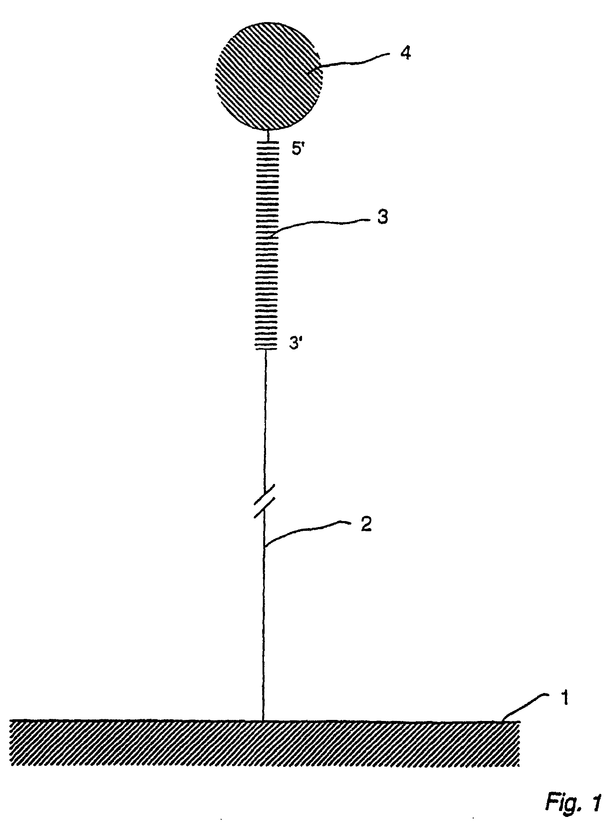

Fig. 1 zeigt eine erste Markierung im nichthybridisierten Zustand. Auf der Oberfläche eines Festkörpers 1, z. B. einer Banknote, ist kovalent ein Spacermolekül 2 gebunden. Das Spacermolekül weist eine Länge von 5 bis 10000 nm, auf. An das freie Ende des Spacermoleküls 2 ist das 3'-Ende einer PNA 3 gebunden. Diese besteht aus etwa 10 bis 50 Basen. An das 5'-Ende der PNA 3 ist ein magnetisierbares Partikel 4 gebunden. Dabei kann es sich um eine Polymer-Gruppe mit einem Durchmesser von 20 bis 200 nm. handeln.

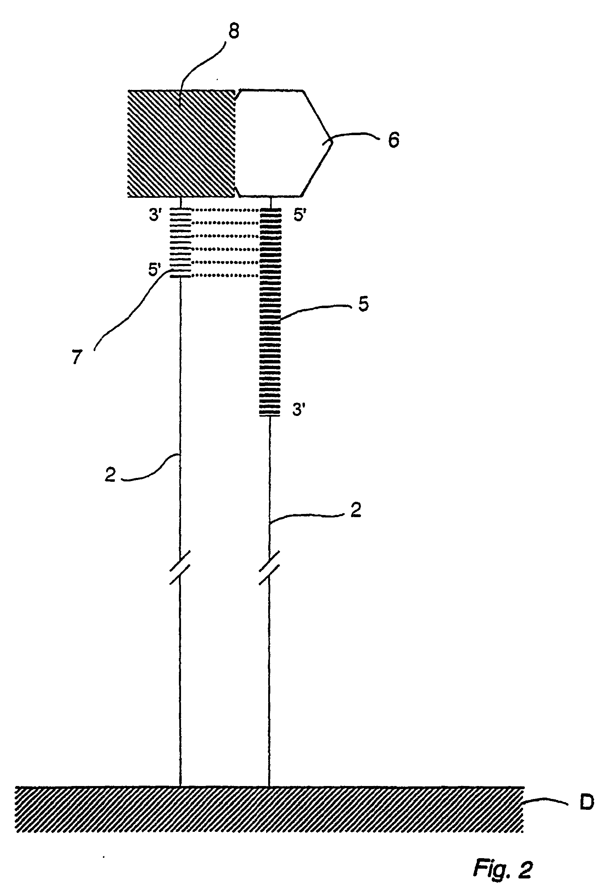

Fig. 2 zeigt eine schematische Teilansicht eines Detektors. An eine aus Kunststoff hergestellte feste Phase D ist über das Spacermolekül 2 eine zweite PNA 5 gebunden. An das 5'-Ende der zweiten PNA 5 ist eine erste fluorophore Gruppe 6 gebunden. Eine dritte PNA 7, die abschnittsweise korrespondierend zur zweiten PNA 5 ausgebildet ist, ist mit ihrem 5'-Ende über ein weiteres Spacermoiekül 2 an die feste Phase D gebunden. Am 3'-Ende der dritten PNA 7 ist ein zweites fluorophores Molekül oder ein Quencher 8 gebunden.

Das erste fluorophore Molekül 6 kann eine Akzeptorgruppe und das zweite fluorophore Molekül eine Donorgruppe sein. Die Akzeptorgruppe kann ein 6-Carboxy-Tetramethyl-Rhodamin und die Donor-Gruppe ein 6-Carboxy-Fluorescein sein. Weitere geeignete Donor-/Akzeptor-Paare sind aus der nachstehenden Tabelle ersichtlich:

| Donor | Akzeptor |
|---|---|
| Fluorescein | Fluorescein |
| Fluorescein | Tetramethylrhodamin |
| IAEDANS = (5-((((2-iodoacyl) amino)ethyl)amino) naphathalene-lsulfonsäure) | Fluorescein |
| EDANS (5-((2-aminomethyl) amino)naphthalen-1-sulfonsäure | DABCYL (4-dimethylaminoazo benzen-4'-sulfoylochlorid) |
| BODIPY FL | BODIPY FL |

Es ist selbstverständlich möglich, dass das erste und das zweite fluorophore Molekül gegeneinander ausgetauscht sind. Nach einem weiteren Ausgestaltungsmerkmal kann das zweite fluorophore Molekül durch einen Quencher 8 ersetzt sein.

Geeignete Quencher/Fluorophor-Paare sind aus der folgenden Tabelle ersichtlich:

| Quencher | Fluorophor |
|---|---|
| DABCYL | Coumarin |
| DABCYL | EDANS |
| DABCYL | Fluorescein |
| DABCYL | Lucifer-Yellow |
| DABCYL | Bodipy |
| DABCYL | Eosin |
| DABCYL | Tetramethylrhodamin |
| DABCYL | Texas-Red |
| DABCYL | Érythrosin |

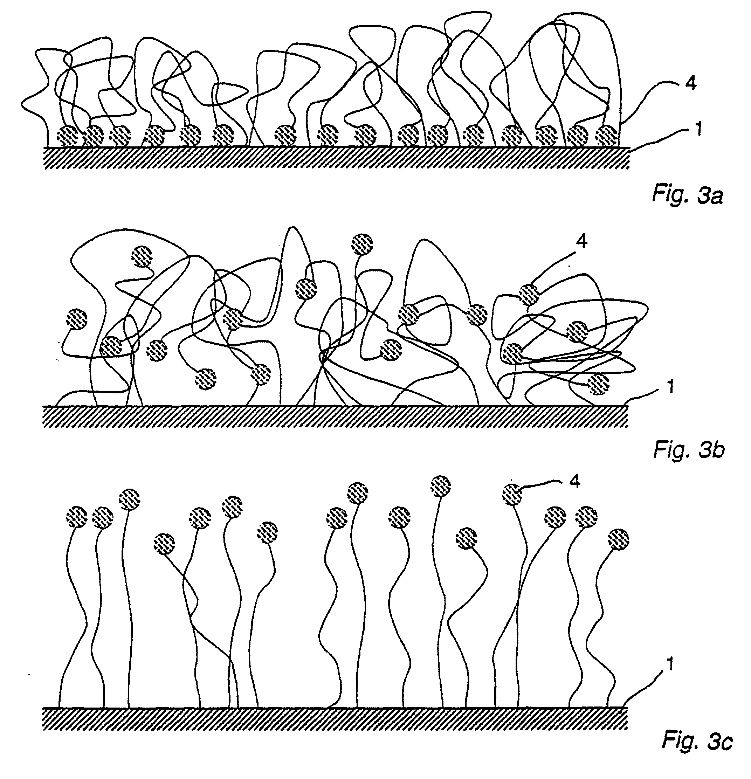

Die Fig. 3 a bis c zeigen das Verhalten der Markierung unter dem Einfluß eines Magnetfelds. Bei Anlegen eines entsprechend orientierten Magnetfelds werden die magnetischen Partikel 4 von der Oberfläche des Festkörpers 1 wegbewegt.

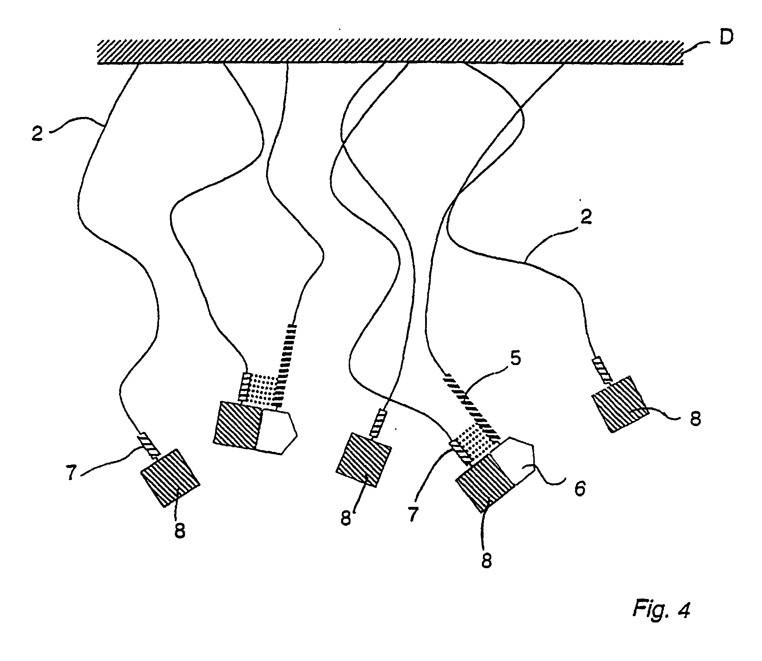

Fig. 4 zeigt nochmals die Oberfläche des Detektors gemäß Fig. 2. Daraus ist ersichtlich, dass an der Oberfläche der festen Phase D mit zweiten fluorophoren Molekülen bzw. Quenchern 8 versehene dritte PNA 7 im Überschuß über Spacermoleküle 2 an die Oberfläche gebunden sind. Damit ist sichergestellt, dass im Falle der Benutzung eines Quenchers 8 - die Fluoreszenz des ersten fluorophoren Moleküls 6 gelöscht wird, so lange der Detektor nicht im Kontakt mit einer ersten korrespondierend zur zweiten PNA 5 ausgebildeten PNA 3 ist.

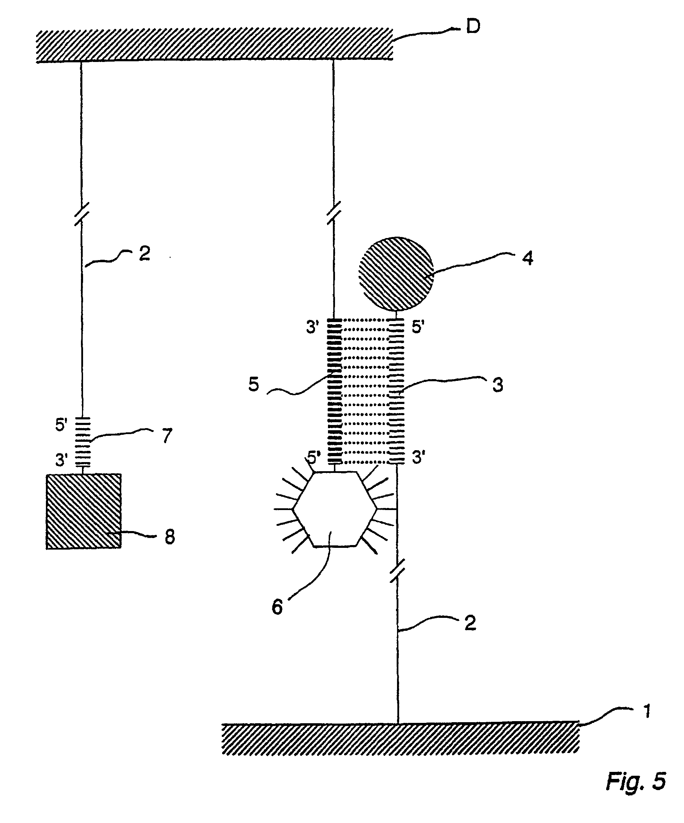

Fig. 5 zeigt die Markierung gemäß Fig. 1 im Kontakt mit dem Detektor gemäß Fig. 3. Die erste PNA 3 hybridisiert mit der zweiten PNA 5 des Detektors. Die dritte PNA 7 ist von der zweiten PNA 5 getrennt. Es besteht keine Wechselwirkung zwischen dem ersten fluorophoren Molekül 6 und Quencher 8 mehr. Dadurch wird eine Fluoreszenzreaktion hervorgerufen. Im Falle der Verwendung eines zweiten fluorophoren Moleküls ist eine geänderte Fluoreszenz beobachtbar. Die Bindung zwischen zweiten PNA 5 und der dritten PNA 7 kann vor dem Inkontaktbringen der Markierung mit dem Detektor durch Temperaturerhöhung gelöst werden. Dazu wird die Markierung beispielsweise mittels eines IR-Laserpulses kurz aufgeheizt.

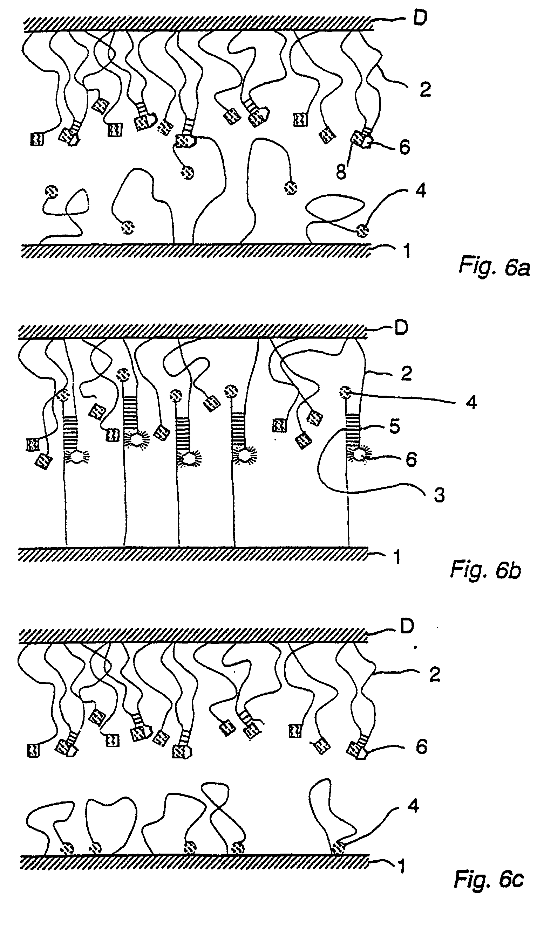

Die Fig. 6 a bis c zeigen nochmals schematisch den Detektionsvorgang. Zunächst wird gemäß Fig. 6a der Detektor in die Nähe der Markierung gebracht. Anschließend wird die Markierung kurzzeitig aufgeheizt und ein Magnetfeld angelegt. Dadurch wird die erste PNA 3 in Richtung der festen Phase D bewegt. Sie hybridisiert mit der zweiten PNA 5, welche durch den Wärmeimpuls von der dritten PNA 7 gelöst worden ist. Es kommt infolge der Hybridisierung zu einer Fluoreszenzreaktion. Nach deren Detektion durch einen geeigneten Fluoreszenz-Detektor wird das Magnetfeld gegebenenfalls umgepolt und damit der Detektionsvorgang beendet.

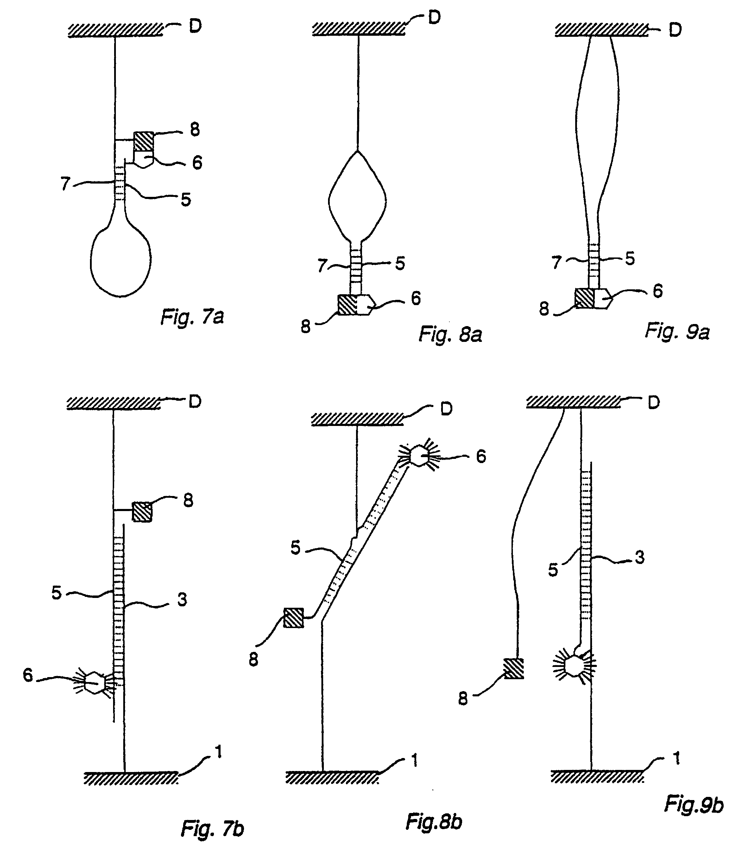

Die Fig. 7 a und b zeigen eine zweite Ausführungsform eines 20 Detektors. Dabei ist die zweite PNA 5 und die dritte PNA 7 an ein und demselben Nukleotidstrang vorgesehen. Durch die Hybridisierung der zweiten PNA 5 mit der dritten PNA 7 bildet sich eine Schleife. Das erste fluorophore Molekül 6 der Quencher 8 bilden eine Wechselwirkung aus. Bei dem in Fig. 8a gezeigten Ausführungsbeispiel ist die zweite PNA 5 an einem Zweig und die dritte PNA 7 an einem anderen Zweig eines verzweigten Moleküls vorgesehen. Fig. 9a zeigt nochmals die vorbeschriebene Ausbildung des Detektors, bei der die zweite PNA 5 und die dritte PNA 7 an zwei separaten Strängen an die feste Phase D gebunden sind.

Die Fig. 7b, 8b und 9b zeigen jeweils den Hybridisierungszustand.

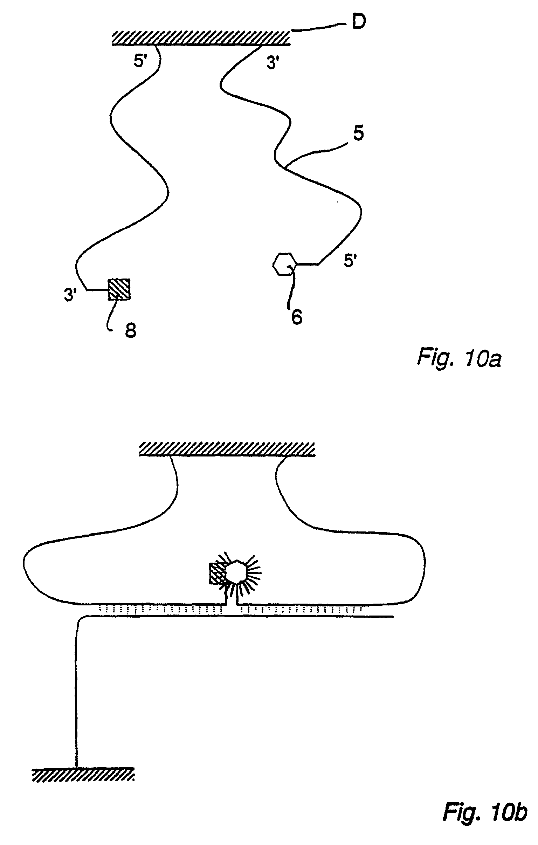

Fig. 10a zeigt ein weiteres Ausführungsbeispiel. Dabei ist die zweite PNA 5 unmittelbar mit ihrem 3'-Ende an die feste Phase D gebunden. Am 5'-Ende befindet sich das erste fluorophore Molekül 6. Die dritte PNA 7 ist mit ihrem 5'-Ende unmittelbar an der festen Phase D gebunden. Am 3'-Ende der dritten PNA 7 befindet sich in diesem Fall ein zweites fluorophores Molekül 8. Im Hybridisierungsfall binden die zweite PNA 5 und die dritte PNA 7 so an die erste PNA 3, dass eine räumliche Beziehung zwischen dem ersten 6 und dem zweiten fluorophoren Molekül 8 entsteht. Es kommt in diesem Ausführungsbeispiel nach dem Förster-Effekt zu einem strahlungslosen Energieübergang vom zweiten 8 auf das erste fluorophore Molekül 6 und damit zu einer verstärkten Fluoreszenzreaktion.

Beispiel 1:

Design und Synthese eines Markierungsnukleotids und eines Detektornukleotids

Als Markierungsmittel wird ein aus 32 Nukleotiden bestehendes Oligonukleotid folgender Sequenz synthetisiert:

$$\text{5'-CCAAGC CTGGAGGGATGATACTTT GCGCTTGG-3'}$$

Als Detektionsmittel wird ein aus 32 Nukleotiden bestehendes Oligonukleotid synthetisiert, das komplementär zu der Sequenz des Markierungsmittels bzw. -nukleotids ist:

$$\text{3'-X-GGTTCG GACCTCCCTACTATGAAACG CGAACC-6FAM-5'}$$

$$\text{X = dT(C2-DABCYL)}$$

Der Syntheseansatz beträgt 0,2 µmol. Die Oligonukleotide werden über HPLC gereinigt.

Die sechs endständigen Nukleotide innerhalb eines Oligonukleotids sind komplementär zueinander. Daher könnten diese Nukleotide eine Rückfaltung ausbilden.

```
                          GACCTCCCT
              3'-X-GGTTCG          A
              5'-6FAM-CCAAGC        C
                          GCAAAGTAT
```

In dieser Rückfaltung ist die 5'-6FAM-Gruppe am Detektornukleotid in unmittelbarer Nachbarschaft zu der 3'-DABCYL-Gruppe. Bei einer entsprechenden Anregung der 6FAM-Gruppe (Absorptionsmaximum 496nm, Emissionsmaximum 516nm kommen), kann es zu einem strahlungslosen Energietransfer der absorbierten Energie auf die DABCYL-Gruppe kommen. Dadurch ist die Fluoreszenz der 6FAM-Gruppe in diesem Zustand stark reduziert.

Bei einer Zusammenführung des Detektormittels bzw. - nukleotids mit dem Markierungsnukleotid kommt es zu einer Hybridisierung der komplementären Sequenzen beider Moleküle:

```
    5'-CCA AGC CTG GAG GGA TGA TAC  TTT GCG CTT  GG-3'
  3'-X-GGT TCG GAC CTC  CCT ACT  ATG AAA CGC GAA CC-6FAM-5'
```

In der hybridisierten Struktur ist die 5'-6FAM von der 3'-X-Gruppe des Detektornukleotids räumlich getrennt. Damit ist ein Energietransfer zwischen der 6FAM und der DABCYL-Gruppe nicht mehr möglich. Bei einer entsprechenden Anregung der 6FAM-Gruppe kommt es dementsprechend zu einer ungestörten Fluoreszenz der 6FAM-Gruppe.

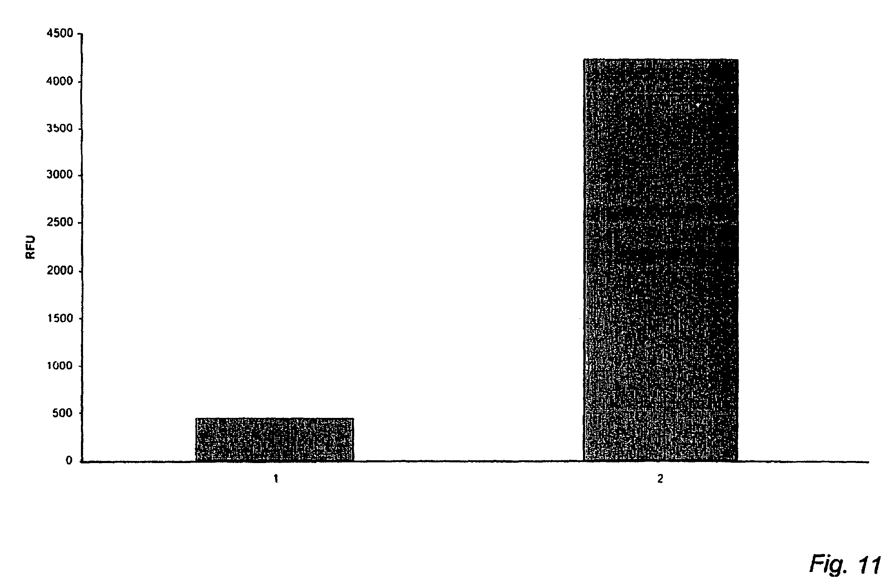

Zum Nachweis eines Fluoreszenz-Energietransfers werden Detektor- und Markierungsnukleotid in 10mM Tris-Cl, 1mM EDTA, pH8 (TE) gelöst. Zu 100µl einer 1µM-Lösung des Detektornukleotids werden das gleiche Volumen einer 2µM-Lösung des Markierungsnukleotids bzw das gleiche Volumen TE zugefügt. Zum Nachweis der Hybridisierung wird die Fluoreszenz der Lösung bei einer Anregung mit 496nm und einer Emission von 516nm bestimmt. Durch die Mischung der Nukleotide steigt die Fluoreszenz um einen Faktor von ca. neun an (Fig. 11). Die Erhöhung der Fluoreszenz zeigt die Aufhebung des intramolekularen Fluoreszenz-Energie-Transfers des Detektornukleotids aufgrund einer Hybridisierung von Detektor- und Markierungsnukleotid an.

Beispiel 2:

Bestimmung der Nachweisempfindlichkeit von Hybriden aus Detektor- und Markierungsnukleotiden

Zur Bestimmung der Nachweisempfindlichkeit von Hybriden aus Detektor- und Markierungsnukleotiden wird die Fluoreszenz einer absteigenden Konzentrationsreihe von Hybriden aus Markierungs- und Detektornukleotid gemessen. Dazu werden Tropfen mit einem Volumen von jeweils 1µl einer 1µM-Lösung des Markierungsnukleotids auf einen Objektträger gegeben. Zu jedem Tropfen wird das gleiche Volumen einer absteigenden Konzentrationsreihe des Detektornukleotids gegeben. Die Konzentrationen an Detektornukleotid betragen 1000, 500, 200, 100, 50, 20, 10, 5, 0 nM. Zur Bestimmung des Hintergrunds werden die gleiche Konzentrationsreihe des Detektornukleotids zu jeweils 1µl TE gegeben. Die Proben werden im Fluoreszenzmikroskop unter der Verwendung eines FAM-spezifischen Filterblocks bei einer 500-fachen Vergrößerung untersucht.

Eine spezifische Fluoreszenz aufgrund der Hybridisierung von Detektor- und Markierungsnukleotid konnte noch nach Zugabe einer Detektornukleotid-Lösung einer Konzentration von 5nM zu der Markierungsnukleotid-Lösung nachgewiesen werden. Ohne Markierungsnukleotid war die Fluoreszenz des Detektornukleotids bis zu einer Konzentration von 50nM sichtbar.

Beispiel 3:

Hybridisierung von Markierungsnukleotid und Detektornukleotid im trockenen Zustand

Zum Nachweis einer Hybridisierung von Markierungs- und Detektornukleotid im trockenen Zustand wird eine 1µM-Lösung des Markierungsnukleotid in TE hergestellt. Der Lösung wird zur Sichtbarmachung des Markierungsnukleotid zusätzlich 4,6-Diamidino-2-phenylindole (DAPI) mit einer Konzentration von 2ng/µl zugefügt. DAPI ist ein Farbstoff, der DNA bindet und im gebundenen Zustand fluoresziert. Die Fluoreszenz von DAPI läßt sich durch entsprechende Filter von der Fluoreszenz der FAM-Gruppe trennen und nachweisen.

1µl der Markierungsnukleotid-Lösung wird mit einer 2µl Pipette in Form von Linien auf einen polierten Objektträger aufgetragen. Die Flüssigkeit wird bei Raumtemperatur eingetrocknet. Fig. 12 a zeigt einen solchen beschichteten Objektträger. Das Markierungsnukleotid ist in Form der hellen Stellen erkennbar. Auf einem zweiten Objektträger wird auf einer Fläche von ca. 40mm$^2$ 5µl einer 20nM-Lösung des Detektornukleotids aufgetragen und ebenfalls bis zur Trocknung bei Raumtemperatur stehengelassen. Danach werden die Objektträger mit den Seiten die Markierungsnukleotide tragenden, aufeinander gelegt und mit einem Anpressdruck von ca. 400N/cm$^2$ für eine Minute zusammen gepreßt. Dann werden die Objektträger im Fluoreszenzmikroskop bei einer 500-fachen Vergrößerung untersucht. Das aufgetragene Markierungsnukleotid kann anhand der DAPI-Färbung nachgewiesen werden. Das Detektornukleotid ist im nicht-hybridisierten Zustand nicht nachweisbar. Im hybridisierten Zustand kann das Detektornukleotid anhand der FAM-spezifischen Fluoreszenz nachgewiesen werden. Die Fluoreszenz des Detektornukleotids ist nur in dem Bereich des aufgetragenen Markierungsnukleotids zu beobachten (Fig. 12 b). Die beobachtete Fluoreszenz des Detektornukleotids im Bereich des aufgetragenen Markierungsnukleotids ist ein Nachweis für die Hybridisierung des Markierungsnukleotids mit dem Detektornukleotid.

Bezugszeichenliste

| 1 | Festkörper |
|---|---|
| 2 | Spacermolekül |
| 3 | erste PNA |
| 4 | magnetische Gruppe |
| 5 | zweite PNA |
| 6 | erstes fluorophores Molekül |
| 7 | dritte PNA |
| 8 | zweites fluorophores Molekül bzw. Quencher |

D    feste Phase

**Patentansprüche**

1.  Verfahren zum Identifizieren einer an einem Festkörper (1) vorgesehenen Markierung, die eine erste mit ihrem einen Ende am Festkörper (1) gebundene Nukleotidsequenz (3) aufweist,
    wobei die erste Nukleotidsequenz (3) mit einem Detektionsmittel in Kontakt gebracht-wird,
    wobei das Detektionsmittel eine feste Phase (D) aufweist, an die eine zur ersten Nukleotidsequenz (3) korrespondierende zweite Nukleotidsequenz (5) mit ihrem ersten Ende gebunden ist und wobei die zweite Nukleotidsequenz (5) am zweiten Ende eine erste fluorophore Gruppe (6) aufweist,
    wobei beim Inkontaktbringen die erste (3) und die zweite Nukleotidsequenz (5) hybridisieren,
    und wobei bei der Hybridisierung der ersten (3) und zweiten Nukleotidsequenz (5) eine Fluoreszenzreaktion ausgelöst wird.

2.  Verfahren nach einem der vorhergehenden Ansprüche, wobei vor oder beim Inkontaktbringen eine Hybridisierung der ersten Nukleotidsequenz (3) mit einer dritten Nukleotidsequenz (7) gelöst wird.

3.  Verfahren nach einem der vorhergehenden Aasprüche, wobei die erste Nukleotidsequenz (5) beim Inkontaktbringen durch Magnetwirkung in Richtung des Detektionsmittels bewegt wird.

4.  Verfahren nach einem der vorhergehenden Ansprüche, wobei vor dem Inkontaktbringen eine die erste Nukleotidsequenz (3) überdeckende Schutzschicht entfernt wird.

5.  Verfahren nach einem der vorhergehenden Ansprüche, wobei vor dem Inkontaktbringen die Markierung auf eine Temperatur von mehr als 50°C erwärmt wird.

6.  Verfahren nach einem der vorhergehenden Ansprüche, wobei nach dem Inkontaktbringen die Markierung auf eine Temperatur von weniger als 50°C abgekühlt wird.

7.  Verfahren nach einem der vorhergebenden Ansprüche, wobei die Hybridisierung zwischen der ersten (3) und der zweiten Nukleotidsequenz (5) unterstützt wird durch die Einwirkung von Licht einer vorgegebenen Wellenlänge.

8.  Vorrichtung zum Identifizieren einer an einem Festkörper (1) vorgesehenen Markierung, die eine erste mit ihrem einen Ende am Festkörper (1) gebundene Nukleotidsequenz (3) aufweist,
    mit einem Detektionsmittel, das eine feste Phase (D) aufweist, an die eine zur ersten Nukleotidsequenz (3) korrespondierende zweite Nukleotidsequenz (5) mit ihrem ersten Ende gebunden ist und am zweiten Ende eine erste fluorophore Gruppe (6) aufweist,
    so dass beim Inkontaktbringen des Detektionsmittels mit der Markierung die erste (3) und die zweite Nukleotidsequenz (5) hybridisieren und bei der Hybridisierung der ersten (3) und zweiten Nukleotidsequenz (5) eine Fluoreszenzreaktion auslösbar ist.

9.  Vorrichtung nach Anspruch 8, wobei ein erstes Ende der ersten Nukleotidsequenz über Spacermoleküle (2) kovalent an den Festkörper (1) gebunden ist.

10. Vorrichtung nach einem der Ansprüche 8 oder 9, wobei an ein zweites Ende der ersten Nukleotidsequenz (3) ein magnetisches Mittel (4) gebunden ist.

**11.** Verrichtung nach einem der Ansprüche 8 bis 10, wobei das Detektionsmittel einen Magneten aufweist.

**12.** Vorrichtung nach einem der Ansprüche 8 bis 11, wobei das erste Ende der zweiten Nukleotidsequenz (5) über Spacermoleküle (2) an die feste Phase (D) gebunden ist.

**13.** Vorrichtung nach einem der Ansprüche 8 bis 12, wobei das Detektionsmittel eine dritte Nukleotidsequenz (7) aufweist, die zumindest abschnittsweise zur ersten (3) oder zweiten Nukleotidsequenz (5) korrespondierend ausgebildet ist.

**14.** Vorrichtung nach Anspruch 13, wobei ein erstes Ende der dritten Mukleotidsequenz (7) über Spacermoleküle (2) an die feste Phase (D) gebunden ist.

**15.** Verrichtung nach einem der Ansprüche 13 oder 14, wobei an einem zweiten Ende der dritten Nukleotidsequenz eine zweite fluorophore Gruppe oder ein Quencher vorgesehen ist.

**16.** Vorrichtung nach einen der Anspriächa 13 bis 15, wobei die zweite und die dritte Nukleotidsequenz bei der Identifizierung im hybridisierten Zustand vorliegen, so dass eine Wachselwirkung zwischen dem ersten fluorophoren Molekül und dem zweiten fluorophoren Molekül bzw. dem Quencher ausbild- oder unterbrechbar ist.

**17.** Vorrichtung nach einem der Ansprüche 13 bis 15, wobei die erste (3), zweite (5) und dritte Nukleotidsequenz (7) aus DNA oder PNA gebildet ist.

**18.** Vorrichtung nach einem der Ansprüche 8 bis 17, wobei auf der Oberfläche der festen Phase eine aus wasserbindenden Substanzen hergestellte Schicht vorgesehen ist.

**19.** Vorrichtung nach einem der Ansprüche 8 bis 18, wobei das Detektormittel eine Lichtquelle und/oder einen Fluoreszenzdetektor aufweist.

**20.** Vorrichtung nach einem der Ansprüche 8 bis 19, wobei das Detektormittel eine Einrichtung zur Erzeugung eines Wärme-Pulses, vorzugsweise eines IR-Lasar-, NMR-, Mikrowellen- oder Ultraschall-Pulses, aufweist.

**Claims**

**1.** A method for identifying a tag provided on a solid (1), which comprises a first nucleotide sequence (3) bound to the solid (1) at its one end,
whereby the first nucleotide sequence (3) is brought into contact with a detection means,
whereby the detection means comprises a solid phase (D), to which a second nucleotide sequence (5) corresponding to the first nucleotide sequence (3) is bonded by its first end and whereby the second nucleotide sequence (5) comprises a first fluorophore group at the second end,
whereby the first (3) and the second nucleotide sequences (5) hybridise upon being brought into contact,
and whereby a fluorescence reaction is triggered upon the hybridisation of the first (3) and second nucleotide sequences (5).

**2.** A method according to one of the preceding Claims, whereby a hybridisation of the first nucleotide sequence (3) with a third nucleotide sequence (7) is released prior to or upon being brought into contact.

**3.** A method according to one of the preceding Claims, whereby the first nucleotide sequence (5) is moved by magnetic action towards the detection means upon being brought into contact.

**4.** A method according to one of the preceding Claims, whereby a protective layer covering the first nucleotide sequence (3) is removed prior to being brought into contact.

**5.** A method according to one of the preceding Claims, whereby the tag is heated to a temperature of more than 50°C prior to being brought into contact.

6. A method according to one of the preceding Claims, whereby the tag is cooled to a temperature of less than 50°C after being brought into contact.

7. A method according to one of the preceding Claims, whereby the hybridisation between the first (3) and the second nucleotide sequence (5) is assisted by the action of light of a predetermined wavelength.

8. A device for identifying a tag provided on a solid (1), which comprises a first nucleotide sequence (3) bonded by its one end to the solid (1),
having a detection means comprising a sold phase (D) to which a second nucleotide sequence (5) corresponding to the first nucleotide sequence (3) is bonded by its first end and comprises a first fluorophore group (6) at the second end,
so that upon bringing the detection means into contact with the tag, the first (3) and the second nucleotide sequence (5) hybridise and a fluorescence reaction can be triggered upon the hybridisation of the first (3) and second nucleotide sequence (5).

9. A device according to Claim 8, wherein a first end of the first nucleotide sequence is covalently bonded to the solid (1) via spacer molecules (2).

10. A device according to one of Claims 8 or 9, wherein a magnetic means (4) is bonded to a second end of the first nucleotide sequence (3).

11. A device according to one of Claims 8 to 10, wherein the detection means comprises a magnet.

12. A device according to one of Claims 8 to 11, wherein the first end of the second nucleotide sequence (5) is bonded to the solid phase (D) via spacer molecules (2).

13. A device according to one of Claims 8 to 12, wherein the detection means comprises a third nucleotide sequence (7), which is constructed corresponding at least in sections to the first (3) or the second nucleotide sequence (5).

14. A device according to Claim 13, wherein a first end of the third nucleotide sequence (7) is bonded via spacer molecules (2) to the solid phase (D).

15. A device according to one of Claims 13 or 14, wherein a second fluorophore group or a quencher is provided at a second end of the third nucleotide sequence.

16. A device according to one of Claims 13 to 15, wherein the second and the third nucleotide sequences are present in the hybridised state during the identification, so that an interaction between the first fluorophore molecule and the second fluorophore molecule or the quencher can be developed or interrupted.

17. A device according to one of Claims 13 to 16, wherein the first (3), second (5) and third (7) nucleotide sequence is made of DNA or PNA.

18. A device according to one of Claims 8 to 17, wherein a layer produced from hydrophilic substances is provided on the surface of the solid phase.

19. A device according to one of Claims 8 to 18, wherein the detector means comprises a light source and/or a fluorescence detector.

20. A device according to one of Claim 8 to 19, wherein the detector means comprises a device for generating a pulse of heat, preferably an IR laser, NMR, microwave or ultrasound pulse.


**Revendications**

1. Procédé d'identification d'une marque prévue sur un solide (1), qui présente une première séquence nucléotidique (3) liée par une de ses extrémités au corps solide (1),
dans lequel la première séquence nucléotidique (3) est amenée au contact d'un agent de détection,
dans lequel l'agent de détection présente une phase solide (D) à laquelle est liée par sa première extrémité une seconde séquence nucléotidique (5) correspondant à la première séquence nucléotidique (3), et dans lequel la seconde séquence nucléotidique (5) présente sur sa deuxième extrémité un groupe fluorophore (6), dans lequel lors de la mise en contact, la première (3) et la deuxième (5) séquences nucléotidiques s'hybrident,

et dans lequel lors de l'hybridation de la première (3) et de la seconde (5) séquences nucléotidiques, une réaction de fluorescence est déclenchée.

2.  Procédé selon la revendication précédente, dans lequel, avant ou pendant la mise en contact, la première séquence nucléotidique (3) est libérée d'une hybridation par une troisième séquence nucléotidique (7).

3.  Procédé selon l'une quelconque des revendications précédentes, dans lequel la première séquence nucléotidique (3) est déplacée en direction de l'agent de détection par l'action d'un aimant lors de la mise en contact.

4.  Procédé selon l'une quelconque des revendications précédentes, dans lequel une couche protectrice recouvrant la première séquence nucléotidique (3) est retirée avant la mise en contact.

5.  Procédé selon l'une quelconque des revendications précédentes, dans lequel la marque est chauffée à une température supérieure à 50 °C avant la mise en contact.

6.  Procédé selon l'une quelconque des revendications précédentes, dans lequel la marque est refroidie à une température inférieure à 50 °C après la mise en contact.

7.  Procédé selon l'une quelconque des revendications précédentes, dans lequel l'hybridation entre la première séquence nucléotidique (3) et la deuxième séquence nucléotidique (5) est assistée par l'action de la lumière d'une longueur d'onde prédéterminée.

8.  Dispositif d'identification d'une marque prévue sur un corps solide (1), qui présente une première séquence nucléotidique (3) liée par une extrémité au corps solide (1),

comportant un agent de détection qui présente une phase solide (D), à laquelle est liée par sa première extrémité une seconde séquence nucléotidique (5) correspondant à la première séquence nucléotidique (3), et présentant sur la deuxième extrémité un premier groupe fluorophore (6),

de sorte que, lors de la mise en contact de l'agent de détection avec la marque, la première (3) et la deuxième (5) séquences nucléotidiques s'hybrident, et lors de l'hybridation de la première (3) et de la deuxième (5) séquences nucléotidiques, une réaction de fluorescence peut être déclenchée.

9.  Dispositif selon la revendication 8, dans lequel une première extrémité de la première séquence nucléotidique est liée par covalence au corps solide (1) par l'intermédiaire de molécules d'espacement (2).

10. Dispositif selon l'une des revendications 8 ou 9, dans lequel un agent magnétique (4) est lié à une deuxième extrémité de la première séquence nucléotidique (3).

11. Dispositif selon l'une des revendications 8 à 10, dans lequel l'agent de détection comprend un aimant.

12. Dispositif selon l'une des revendications 8 à 11, dans lequel la première extrémité de la deuxième séquence nucléotidique (5) est liée à la phase solide (D) par l'intermédiaire de molécules d'espacement (2).

13. Dispositif selon l'une des revendications 8 à 12, dans lequel l'agent de détection présente une troisième séquence nucléotidique (7) qui est conçue de manière à correspondre au moins par segments à la première séquence nucléotidique (3) ou à la deuxième séquence nucléotidique (5).

14. Dispositif selon la revendication 13, dans lequel une première extrémité de la troisième séquence nucléotidique (7) est liée à la phase solide (D) par l'intermédiaire de molécules d'espacement (2).

15. Dispositif selon l'une des revendications 13 ou 14, dans lequel un deuxième groupe fluorophore ou un agent d'extinction est prévu sur une deuxième extrémité de la troisième séquence nucléotidique.

16. Dispositif selon l'une des revendications 13 à 15, dans lequel la deuxième séquence nucléotidique et la troisième séquence nucléotidique sont présentes à l'état hybridé lors de l'identification, de telle sorte qu'une interaction peut se produire ou être interrompue entre la première molécule fluorophore et la deuxième molécule fluorophore ou l'agent d'extinction.

**17.** Dispositif selon l'une des revendications 13 à 16, dans lequel la première séquence nucléotidique (3), la deuxième séquence nucléotidique (5) et la troisième séquence nucléotidique (7) sont formées à partir d'ADN ou d'ANP.

**18.** Dispositif selon l'une des revendications 8 à 17, dans lequel une couche produite à partir de substances se liant à l'eau est prévue sur la surface de la phase solide.

**19.** Dispositif selon l'une des revendications 8 à 18, dans lequel l'agent de détection présente une source de lumière et/ou un détecteur de fluorescence.

**20.** Dispositif selon l'une des revendications 8 à 19, dans lequel l'agent de détection présente une unité destinée à la production d'une impulsion thermique, de préférence d'une impulsion à laser IR, RMN, hyperfréquence ou ultra-sonique.

*Fig. 1*

*Fig. 2*

Fig. 3a

Fig. 3b

Fig. 3c

Fig. 4

*Fig. 5*

Fig. 6a

Fig. 6b

Fig. 6c

Fig. 7a

Fig. 8a

Fig. 9a

Fig. 7b

Fig.8b

Fig.9b

Fig. 10a

Fig. 10b

Fig. 11

*Fig. 12 a*

*Fig. 12 b*